# EUROPÄISCHE PATENTSCHRIFT

(11) **EP 0 769 020 B1**
(45) Veröffentlichungstag und Bekanntmachung des Hinweises auf die Patenterteilung: **01.03.2000**
(21) Anmeldenummer: 95924273.6
(22) Anmeldetag: 19.06.1995
(51) Int. Cl.: C07K 14/54, A61K 38/20

(54) **NEUE hIL-4-MUTANTENPROTEINE ALS ANTAGONISTEN ODER PARTIELLE AGONISTEN DES HUMANEN INTERLEUKIN 4**
NEW hIL-4 MUTANT PROTEINS USED AS ANTAGONISTS OR PARTIAL AGONISTS OF HUMAN INTERLEUKIN 4
NOUVELLES PROTEINES MUTANTES DE L'IL-4 HUMAINE UTILISEES COMME ANTAGONISTES OU AGONISTES PARTIELS DE L'INTERLEUKINE HUMAINE 4

(30) Priorität: 01.07.1994 DE 4423131
(43) Veröffentlichungstag der Anmeldung: 23.04.1997
(73) Patentinhaber: BAYER AG, 51368 Leverkusen (DE)
(72) Erfinder: WILD, Hanno, Orange, CT 06477 (US); HANKO, Rudolf, D-40237 Düsseldorf (DE); DÖRSCHUG, Michael, D-42579 Heiligenhaus (DE); HÖRLEIN, Hans-Dietrich, D-42113 Wuppertal (DE); BEUNINK, Jürgen, D-42329 Wuppertal (DE); APELER, Heiner, D-42115 Wuppertal (DE); WEHLMANN, Hermann, D-42349 Wuppertal (DE); SEBALD, Walter, D-97074 Würzburg (DE)
(86) Internationale Anmeldenummer: EP9502358
(87) Internationale Veröffentlichungsnummer: WO9601274

(56) Entgegenhaltungen:
- WO-A-88/04667
- WO-A-93/10235
- US-A- 5 298 410
- BIOLOGICAL CHEMISTRY HOPPE-SEYLER, Bd. 373, Nr. 9, September 1992 Seiten 789-790, N. KRUSE ET AL 'Mutational analysis of human interleukin-4: identification of crucial amino acids for receptor binding and generation of a high affinity antagonist'
- EMBO JOURNAL, Bd. 11, Nr. 9, September 1992 EYNSHAM, OXFORD GB, Seiten 3237-3244, N. KRUSE ET AL 'Conversion of human interleukin-4 into a high affinity antagonist by a single amino acid replacement'

## Beschreibung

Die vorliegende Erfindung betrifft neue hIL-4-Mutantenproteine, Verfahren zu ihrer Herstellung, sowie ihre Verwendung als Arzneimittel, insbesondere bei überschießenden fehlgesteuerten Immunreaktionen und Autoimmunerkrankungen.

Aus der PCT WO 93/10235 sind bereits therapeutische Mittel, die Antagonisten oder partielle Agonisten des hIL-4 sind oder diese enthalten, wobei die Antagonisten oder partiellen Agonisten hIL-4-Mutantenproteine sind, bekannt.

Humanes Interleukin-4 (hIL-4) ist eines unter den zahlreichen Cytokinen, die die Proliferation, die Reifung, das Überleben und die Differenzierung lymphoider und myeloischer Zellen induzieren und koordinieren. Insbesondere ist hIL-4 an der IgE-mediierten Immunreaktion beteiligt und beschleunigt direkt die Proliferation von Thymocyten und aktivierten T-Zellen. Man konnte ein hochaffines IL-4-Rezeptorprotein mit Mr 140 000 identifizieren, welches gemäß seiner cDNA-Sequenz aus 800 Aminosäureresten besteht. Dieses gehört zu einer kürzlich beschriebenen Gruppe von Rezeptoren, die man als Hämatopoietin-Rezeptor-Superfamilie bezeichnet.

Die Aminosäuresequenz des reifen IL-4 besteht aus 129 Resten, wenn man die klonierte cDNA zugrundelegt. Die cDNA ist in E.coli und Hefe exprimiert worden. Aus diesen Quellen kann rekombinantes IL-4 mit hoher biologischer Aktivität gewonnen werden.

In jüngster Zeit ist bereits ein monoclonaler Antikörper bekannt geworden, der gegenüber dem menschlichen Interleukin-4 antagonistische Eigenschaften aufweist. Dieser Antikörper enthält ein Fab-Fragment und wird von einer Mensch-Mensch-Hybridomazellinie produziert. Auch eine Hybridomazellinie aus Milzzellen einer gegen (nicht-)glycosyliertes menschliches IL-4 immunisierten Ratte produziert monoclonale Antikörper gegen hIL-4.

Die Rolle des Interleukin 4 bei allergischen Prozessen läßt hoffen, daß Substanzen, die Interleukin-4 vermittelte Prozesse inhibieren oder mit dem hIL-4 konkurrieren, die krankheitsauslösende Reaktionskette unterbrechen.

In DE 41 37 333 A1 sind hIL-4-Mutantenproteine beschrieben, bei denen an einer oder mehreren der Positionen 120, 121, 122, 123, 124, 125, 126, 127 oder 128 die dort natürlicherweise im Wildtyp auftretende(n) Aminosäure(n) gegen eine bzw. mehrere andere der möglichen natürlichen Aminosäuren ausgetauscht ist. Diese hIL-4-Mutantenproteine sind Antagonisten oder partielle Agonisten des humanen IL-4.

Die vorliegende Erfindung betrifft nun neue hIL-4-Mutantenproteine, die Antagonisten oder partielle Agonisten des humanen Interleukin-4 sind, in welchen zusätzlich zu dem/den Austausch/en an den Positionen 121, 124 oder 125 weitere Modifikationen des hIL-4-Proteins durchgeführt worden sind. Diese Modifikationen werden durchgeführt, um die Stabilität des hIL-4-Mutantenproteins zu erhöhen, um die biologische Halbwertszeit zu verlängern oder um das Herstellungs- und Reiningungsverfahren zu erleichtern.

Dazu werden an einer oder mehreren Positionen, die dort natürlicherweise im Wildtyp auftretenden Aminosäuren deletiert bzw durch andere Aminosäuren ersetzt, oder es werden zusätzliche Aminosäuren - auch an C- oder N-Terminus - eingefügt, oder eine oder mehrere der Aminosäuren werden mit verschiedenen Nicht-Protein-Polymeren, wie z.B. Polyethylenglykol und dessen Derivaten- oder durch Glycosylreste substituiert.

Aminosäuren stehen im Rahmen der Erfindung im allgemeinen für
- Ala: L-Alanin
- Arg: L-Arginin
- Asn: L-Asparagin
- Asp: L-Asparaginsäure
- Cys: L-Cystein
- GIn: L-Glutamin
- Glu: L-Glutaminsäure
- Gly: L-Glycin
- His: L-Histidin
- Ile: L-Isoleucin
- Leu: L-Leucin
- Lys: L-Lysin
- Met: L-Methionin
- Pro: L-Prolin
- Phe: L-Phenylalanin
- Ser: L-Serin
- Thr: L-Threonin
- Trp: L-Tryptophan
- Tyr: L-Tyrosin
- Val: L-Valin,
wobei zur Vereinfachung die Konfigurationsbezeichnung unterbleiben kann.

Unter Nicht-Protein-Polymere versteht man z.B. Polyethylenglycol, Polypropylenglycol oder Polyoxyalkylene, wie sie in den US-Patenten Nr. 4.640.835, 4.496.689, 4.301.144, 4.670.417, 4.791.192 oder 4.179.337 beschrieben sind.

Unter Glycosylierung versteht man die Verknüpfung eines Kohlenhydratgerüstes an die Seitenkette eines Asparaginrestes ("N-Glycosylierung") oder die Kopplung eines Zuckers, bevorzugt N-Acetylgalaktosamin, Galaktose oder Xylose an Serin, Threonin, 4-Hydroxyprolin oder 5-Hydroxylysin (O-Glycosylierung).

Bevorzugt sind hIL-4 Mutantenproteine, bei denen die Aminosäure 124 (Tyrosin), die Aminosäuren 121 (Arginin) und Aminosäure 125 (Serin) in beliebiger Kombination gegen eine der möglichen natürlichen Aminosäuren ausgetauscht sind, und bei welchen zusätzlich der N- und/oder C-terminus des Moleküls modifiziert ist und/oder ein oder mehrere Polyethylenglycol-Moleküle kovalent an das Molekül gebunden sind und/oder im Molekül vorhandene Glycosylierungsstellen teilweise oder vollständig deletiert sind.

Besonders bevorzugte Ausführungen aus dieser Gruppe sind Muteine, bei denen die Aminosäure 124 (Tyrosin), die Aminosäuren 121 (Arginin) und Aminosäure 125 (Serin) in beliebiger Kombination gegen Asparaginsäure oder Gutaminsäure ausgetauscht sind, und bei welchen zusätzlich der N- und/oder C-terminus des Moleküls modifiziert ist und/oder ein oder mehrere Polyethylenglycol-Moleküle kovalent an das Molekül gebunden sind und/oder im Molekül vorhandene Glycosylierungsstellen teilweise oder vollständig deletiert sind.

Bevorzugte Ausführungsform ist ebenfalls der Austausch der Aminosäure 121 (Arginin) und 125 (Serin) gegen eine natürlich vorkommende Aminosäure, bevorzugt Asparaginsäure oder Glutaminsäure, und bei welchen zusätzlich der N- und/oder C-terminus des Moleküls modifiziert ist und/oder ein oder mehrere Polyethylenglycol-Moleküle kovalent an das Molekül gebunden sind und/oder im Molekül vorhandene Glycosylierungsstellen teilweise oder vollständig deletiert sind.

Besonders bevorzugt sind weiterhin hIL-4 Mutantenproteine, in welchen die Aminosäure 124 (Tyrosin) gegen eine natürlich vokommende Aminosäure und 0 bis eine weitere Aminosäure an den Positionen 121 und/oder 125 gegen eine andere der möglichen Aminosäuren ausgetauscht sind, und bei welchen zusätzlich der N- und/oder C-terminus des Moleküls modifiziert ist und/oder ein oder mehrere Polyethylenglycol-Moleküle kovalent an das Molekül gebunden sind und/oder im Molekül vorhandene Glycosylierungsstellen teilweise oder vollständig deletiert sind.

Aus dieser Gruppe besonders bevorzugt sind hIL-4 Mutantenproteine, in welchen die Aminosäure 124 (Tyrosin) gegen Asparaginsäure oder Glutaminsäure und die Position 121 gegen eine andere der möglichen Aminosäuren, bevorzugt Asparaginsäure oder Glutaminsäure ausgetauscht sind, und bei welchen zusätzlich der N- und/oder C-terminus des Moleküls modifiziert ist und/oder ein oder mehrere Polyethylenglycol-Moleküle kovalent an das Molekül gebunden sind und/oder im Molekül vorhandene Glycosylierungsstellen teilweise oder vollständig deletiert sind.

Bevorzugte Ausführungen der N-terminalen Modifikation für oben genannte Beispiele ist die Insertion einer Aminosäure, bevorzugt Ala, Gly, Pro, Ser, Thr, Val, besonders bevorzugt Ala, zwischen dem N-terminalen Methionin und dem natürlichen N-terminus des hIL-4 Mutantenproteins.

Beispiele für ein derartig exprimiertes Produkt sind:
Ala(-1)-Tyr(124)Asp
Ala(-1)-Arg(121)Asp-Tyr(124)Asp
Ala(-)-Arg(121)Asp-Tyr(124)Asp-Ser(125)Asp
Ala(-)-Tyr(124)Asp-Ser(125)Asp
Ala(-1)-Arg(121)Asp-Ser(125)Asp

Bevorzugte Ausführung der Deletierung von Glycosylierungsstellen in den oben genannten Ausführungen sind die Austausche von Asparagin in Position 38 gegen eine andere natürlich vorkommende Aminosäure, bevorzugt Asparaginsäure und/ oder Asparagin in Position 105 gegen eine andere natürlich vorkommende Aminosäure, bevorzugt Asparaginsäure.

Beispiele für derartig exprimierte Produkte sind:
Asn(38)Asp-Asn(105)Asp-Tyr(124)Asp
Asn(38)Asp-Asn(105)Asp-Arg(121)Asp-Tyr(124)Asp
Asn(38)Asp-Asn(105)Asp-Arg(121)Asp-Tyr(124)Asp-Ser(125)Asp
Asn(38)Asp-Asn(105)Asp-Tyr(124)Asp-Ser(125)Asp
Asn(38)Asp-Asn(105)Asp-Arg(121)Asp-Ser(125)Asp

hIL-4 läßt sich als rekombinantes Protein (rhIL-4) gentechnisch, z.B. in E.coli, erzeugen. Das dabei gebildete Protein läßt sich solubilisieren, renaturieren und isolieren. Das rhIL-4 besitzt dann eine hohe spezifische biologische Aktivität, die z.B. durch Messung der DNA-Synthese/Proliferation von aktivierten T-Zellen oder der CD23 Expression von aktivierten B-Zellen bestimmt werden kann [vgl. Kruse, N. et al. (1991) FEBS Lett. 286, 58-60; Kikutani, H. et al. (1986) Cell 47, 657-665].

Zur Beschaffung von cDNA, die einen DNA-Bereich umfaßt, der den maturen Bereich von hIL-4 kodiert, oder die den maturen Bereich von hIL-4 kodiert, wird auf Yokota, T., Otsuka, T., Mosmann, T., Banchereau, J., DeFrance, T., Blanchard, D., De Vries, J.E., Lee, F. and Arai, K.I. (1986) Proc. Natl. Acad. Sci, USA 83, 5894-5898 und die dort angeführte Literatur verwiesen. Im vorliegenden Zusammenhang werden unter "cDNA, die den maturen Bereich von hIL-4 kodiert" auch cDNA's verstanden, die bei etwa gleicher Anzahl von Basenpaaren Mutanten der konkret im genannten Stand der Technik angegebenen cDNA darstellen, sofern die damit vorzusehenden hIL-4-Muteine ebenfalls Antagonisten oder partielle Agonisten sind.

Hinsichtlich der Durchnumerierung des den maturen Bereich von hIL-4 kodierenden DNA-Bereiches wird hier Garr. C et al., Biochemistry 1991, 30, 1515-1523 gefolgt.

cDNA, die den maturen Bereich von hIL-4 kodiert, kann durch das Ausschneiden eines EcoRV/BamHI-Fragment aus einer gentechnologisch hergestellten cDNA (z.B. von Britisch Bio-Technology Ltd., Oxford, England) gewonnen werden. Das DNA-Fragment wird unter Zusatz von synthetischen Oligonucleotiden, z.B. 5'-CATGCACAAGTGCGAT und 5'-ATCGCACTTGTG, welche die ersten 4 Aminosäure-Codons von Interleukin-4 und zusätzlich das Codon für das Start-Methionin enthalten, in einen Expressionsvektor integriert, beispielsweise zwischen die Ncol- und BamHI-Schnittstellen des Expressionsvektors pR^{TS}pRC 109 [vgl. Weigel, U., Meyer, M. und Sebald W. (1989) Eur. J. Biochem. 180, 295-300].

Aminosäuresequenz-Varianten von IL-4 werden durch Einführung geeigneter veränderter Nukleotide in die IL-4-kodierende DNA oder durch In-vitro-Synthese der gewünschten IL-4-Form erzeugt. Zu solchen Varianten gehören z.B. Deletionen oder Insertionen oder Substitutionen von Resten innerhalb der IL-4-Aminosäuresequenz. Dabei ist zur Erzielung des Endkonstrukts jede Kombination aus Deletion, Insertion und Substitution zulässig, vorausgesetzt daß das Endkonstrukt die gewünschten Merkmale aufweist. Die Aminosäureveränderungen können auch die post-translationale Prozessierung von IL-4 verändern: z.B. können durch Insertion, Deletion oder anderweitige Beeinflussung der Leader-Sequenz des nativen IL-4 die Anzahl oder die Positionen der Glycosylierungsstellen, die Eigenschaften der Membranverankerung und/oder die intrazelluläre Lokalisation von IL-4 verändert werden.

Bei der Konstruktion von Aminosäuresequenz-Varianten von IL-4 hängen die Lokalisation der Mutationsstelle und die Art der Mutation von der (den) zu verändernden Eigenschaft(en) von IL-4 ab. Die Mutationsstellen können einzeln oder in Reihe verändert werden, so z.B. durch (1) Substitution zunächst mit konservativ ausgewählten Aminosäuren und danach mit radikaleren Wahlmöglichkeiten in Abhängigkeit von den erzielten Ergebnissen, (2) Deletion des Target-Rests oder (3) Insertion von Resten in der Nähe der lokalisierten Stelle.

Eine geeignete Methode zur Identifizierung bestimmter IL-4-Reste oder -Regionen als bevorzugte Mutagenesestellen ist die von Cunningham und Wells (Science, 244: 1081-1085, 1989) beschriebene "Alanin-Scanning-Mutagenese". Dabei wird ein Rest oder eine Gruppe von Target-Resten identifiziert (z.B. geladene Reste wie Arg, Asp, His, Lys und Glu) und durch eine neutrale oder negativ geladene Aminosäure (am besten Alanin oder Polyalanin) ersetzt, um so die Interaktion der Aminosäuren mit dem benachbarten wässrigen Umfeld innerhalb oder außerhalb der Zelle zu beeinflussen. Die Bereiche, die auf die Substitutionen funktionell empfindlich reagieren, werden danach durch die Einführung weiterer oder anderer Varianten an den oder für die Substitionsstellen aufgearbeitet. Dies bedeutet, daß zwar die Stelle für die Einführung einer Aminosäuresequenz-Veränderung vorbestimmt ist, aber die Art der Veränderung per se nicht vorbestimmt sein muß.

Um also die Ausführung einer Mutation an einer bestimmten Stelle zu optimieren, kann am Target-Codon oder an der Target-Region das Verfahren des "Ala-Scanning" oder der Zufalls-Mutagenese durchgeführt werden, wobei die exprimierten IL-4-Varianten auf die optimale Kombination im Interesse der gewünschten Eigenschaft überprüft werden.

Bei der Konstruktion der Aminosäuresequenz-Varianten gibt es also zwei Hauptvariable: die Stelle der Mutation und die Art der Mutation.

Die Größe der Deletionen in einer Aminosäuresequenz beträgt in der Regel etwa 1 bis 30 Reste, bevorzugt etwa 1 bis 10 Reste, und sind im Normalfall hintereinanderliegend. Im Normalfall betreffen die Deletionen unmittelbar nebeneinander liegende Aminosäurereste.

Die Zahl der aufeinanderfolgenden Deletionen wird so gewählt, daß die Tertiärstruktur von IL-4 im betroffenen Bereich, z.B. Cystein-Crosslinking, Beta-Faltblatt-Struktur oder Alpha-Helix, erhalten bleibt.

Zu den Insertionen in einer Aminosäuresequenz gehören amino- und/oder carboxyl-terminale Fusionen mit einer Länge von einem einzigen Rest bis zu Polypeptiden mit 100 oder mehr Resten sowie innerhalb einer Sequenz liegende Insertionen von einzelnen oder mehreren Aminosäure-Resten. Innerhalb einer Sequenz liegende Insertionen (d.h. Insertionen innerhalb der IL-4-Sequenz) können i.a. etwa 1 bis 10 Reste, vorzugsweise 1 bis 5 und optimalerweise 1 bis 3 Reste, umfassen. Beispiele für terminale Insertionen sind IL-4 mit einem N-terminalen Methionylrest, ein Artefakt der direkten Expression von IL-4 in einer rekombinanten Bakterienzellkultur, die Insertion einer oder mehrerer zusätzlicher Aminosäuren zwischen Methionin und dem natürlichen N-Terminus zur besseren Abspaltung des Methionins, z.B. durch bakterieneigene Proteasen, und die Fusion einer heterologen N-terminalen Signalsequenz an den N-Terminus des IL-4-Moleküls zur Förderung der Sekretion von reifem IL-4 aus den rekombinanten Wirtszellen bzw. die Fusion von Polyaminosäuren, z.B Polyhistidin, zur leichteren Isolierung von IL-4. Diese Signalsequenzen werden in der Regel aus den vorgesehenen Wirtszellarten ausgewählt und sind diesen daher homolog. Geeignete Sequenzen sind z.B ompA, ompT, phoA, molE, amp oder pelB für E. coli-, der Alpha-Faktor, Amylase, Invertase, Killer-Toxin sowie Mellitin-Pre-Pro-Peptid für Hefe- und virale Signale wie Herpes gD für Säugerzellen.

Eine weiter hevorzugte Signalsequenz ist die natürliche Signal sequenz des Inter-leukin-4.

Besonders bevorzugt sind die Signalsequenzen, die vom Expressionsorganismus selber abgespalten werden, so daß das Interleukin-4 Mutantenprotein den natürlichen N-terminus aufweist.

Bevorzugte Expressionsprodukte nach Abspaltung der Signalsequenz sind:
Tyr(124)Asp
Arg(121)Asp-Tyr(124)Asp
Arg(121)Asp-Tyr(124)Asp-Ser(125)Asp
Tyr(124)Asp-Ser(125)Asp
Arg(121)Asp-Ser(125)Asp

Zu den weiteren Insertions-Varianten von IL-4 gehören die Fusion immunogener Polypeptide an den N- oder C-Terminus von IL-4, z.B. bakterielle Polypeptide wie Beta-Lactamase oder ein durch den E. coli-trp-Locus kodiertes Enzym oder ein Hefeprotein, sowie C-terminale Fusionen mit Proteinen mit langer Halbwertzeit wie z.B. immunglobulinkonstante Regionen (oder andere Immunglobulin-Regionen), Albumin oder Ferritin - vgl. Beschreibung in WO 89/02922 (veröffentlicht am 6. April 1989).

Eine weitere Gruppe von Varianten sind diejenigen mit Aminosäuresubstitution. Bei diesen Varianten ist mindestens ein Aminosäurerest im IL-4-Molekül durch einen anderen Rest ersetzt.

Die natürlich vorkommenden Reste werden auf der Grundlage gemeinsamer Seitenketten-Charakteristika in Klassen gegliedert:
1) hydrophob: Met, Ala, Val, Leu, Ile;
2) neutral hydrophil: Cys, Ser, Thr;
3) sauer: Asp, Glu;
4) basisch: Asn, Gln, His, Lys, Arg;
5) Reste mit Einfluß auf die Kettenorientierung: Gly,
   Pro; und
6) aromatisch: Trp, Tyr, Phe.

Nichtkonservative Substitutionen beinhalten den Austausch eines Vertreters einer dieser Klassen gegen den einer anderen.

Die Aminosäuresubstitution findet u.a. Anwendung bei der Änderung des nativen Glycosylierungsmusters des Polypeptids. "Änderung" heißt Deletion eines oder mehrerer der Kohlenhydratgerüste im nativen IL-4 und/oder Hinzufügung einer oder mehrerer Glycosylierungsstellen, die im nativen IL-4 nicht vorhanden sind.

Die Glycosylierung der Polypetide ist normalerweise entweder N-verknüpft oder O-verknüpft "N-verknüpft" bezieht sich auf die Kopplung des Kohlenhydratgerüsts an die Seitenkette eines Asparaginrestes. Die tri-Peptid-Sequenzen Asparagin-X-Serin und Asparagin-X-Threonin, wobei X jede Aminosäure mit Ausnahme von Prolin sein kann, sind die Erkennungssequenzen für die enzymatische Kopplung des Kohlenhydratgerüsts an die Asparagin-Seitenkette. Die Anwesenheit einer dieser tri-Peptid-Sequenzen in einem Polypeptid schafft demzufolge eine potentielle Glykosylierungsstelle.

"O-verknüpft" bezieht sich auf die Kopplung eines der Zucker N-Acetylgalactosamin, Galaktose oder Xylose an eine Hydroxyaminosäure, i.a. Serin oder Threonin, obwohl auch 4-Hydroxyprolin oder 5-Hydroxylysin verwendet werden können.

Die Hinzufügung von Glykosylierungsstellen an IL-4 erfolgt problemlos durch Änderung der Aminosäuresequenz derart, daß diese eine oder mehrere der oben beschriebenen tri-Peptid-Sequenzen (für N-verknüpfte Glykosylierungsstellen) enthält. Die Änderung kann ebenso durch Addition oder Substitution von einem oder mehreren Serin- oder Threoninresten an die native IL-4-Sequenz (für O-verknüpfte Glykosylierungsstellen) erfolgen. Im Sinne eines einfacheren Vorgehens wird die IL-4-Aminosäuresequenz vorzugsweise über Änderungen auf der Ebene der DNA geändert, insbesondere über eine Mutation der IL-4-kodierenden DNA an vorher ausgewählten Basen, so daß Codons erzeugt werden, die in die gewünschten Aminosäuren translatiert werden. Analog wird bei der gewünschten Deletion des Kohlenhydratgerüstes eine oder mehrere der vorhandenen tri-Peptid-Sequenzen (für N-verknüpfte Glycosylierungen) durch Substitution oder Deletion des ganzen oder Teilen des tri-Peptids modifiziert Im Falle von O-Glycosylierungsstellen kann das Kohlenhydratgerüst durch Substitution oder Deletion der entsprechenden Aminosäure deletiert werden.

Eine weitere Möglichkeit zur Erhöhung der Anzahl der Kohlenhydratgerüste in IL-4 liegt in der chemischen oder enzymatischen Ankopplung von Glykosiden an das Polypeptid. Diese Verfahren sind insofern von Vorteil, als daß sie die Herstellung des Polypeptids nicht in einer Wirtszelle mit Glykosylierungsmöglichkeiten für die N- und O-verknüpfte Glykosylierung erfordern. Je nach dem verwendeten Kopplungsmechanismus kann (können) der (die) Zucker mit (a) Arginin und Histidin, (b) freien Carboxylgruppen, (c) freien Sulfhydrylgruppen wie denen von Cystein, (d) freien Hydroxylgruppen wie denen von Serin, Threonin oder Hydroxyprolin, (e) aromatischen Resten wie denen von Phenylalanin, Tyrosin oder Tryptophan oder (f) der Amidogruppe von Glutamin verknüpft werden. Diese Methoden werden in WO 87/05330, veröffentlicht am 11. September 1987, und bei Aplin und Wriston (CRC Crit. Rev. Biochem., S. 259-306 [1981]) beschrieben.

Zur Entfernung der im nativen IL-4 vorkommenden Kohlenhydratgerüste können neben der oben erwähnten Methode auch chemische oder enzymatische Mittel angewandt werden. Bei der chemischen Deglykosylierung ist die Exposition des Polypeptids gegenüber der Verbindung Trifluormethansulfonsäure oder einer äquivalenten Verbindung erforderlich. Diese Behandlung führt zur Abspaltung der meisten oder aller Zucker mit Ausnahme des verknüpfenden Zuckers (N-Acetylglucosamin oder N-Acetylgalaktosamin), läßt aber das Polypeptid intakt. Die chemische Deglykosylierung wird von Hakkimuddin et al., Arch. Biochem. Biophys., 259:52 (1987) und von Edge et al., Anal. Biochem., 118:131 (1981) beschrieben. Die enzymatische Abspaltung von Kohlenhydratgerüsten in den Polypeptiden ist durch die Verwendung einer Reihe von Endo- und Exoglykosidasen - beschrieben von Thotakura et al. (Meth. Enzymol., 138:350 [1987]) - zu erreichen.

Die Glykosylierung an den potentiellen Glykosylierungsstellen kann durch Verwendung der Verbindung Tunicamycin - beschrieben von Duskin et al. (J. Biol. Chem., 257:3105 [1982]) - verhindert werden. Tunicamycin blockiert die Bildung von Protein-N-Glykosid-Verknüpfungen.

Ein weiterer Typ einer kovalenten Modifikation von IL-4 schließt die Verknüpfung von IL-4 an verschiedene Nicht-Protein-Polymere, z.B. Polyethylenglykol, Polypropylenglykol oder Polyoxyalkylene, in der Art und Weise ein, wie sie in den US-Patenten Nr. 4,640,835; 4,496,689; 4,301,144; 4,670,417; 4,791,192 oder 4,179,337 beschrieben wird.

Das zur Quervernetzung verwendete Agens, der Substitutionsgrad und die Reaktionsbedingungen wird durch Experimente mit bifunktionellen Agentien ausgewählt, vorzugsweise mit einer Reihe von Reagenzien, von denen jedes mit einer anderen Seitenkette reagiert.

Eine bevorzugt genutzte Möglichkeit zur Verbesserung der Halbwertzeit von in vivo zirkulierenden Proteinen ist dessen Konjugation an ein Polymer, das ihm eine längere Halbwertzeit verleiht. So hat sich z.B. die Konjugation von Polyethylenglykol (PEG) an Cl-NH als eine ausgezeichnete Möglichkeit zur Verlängerung der Halbwertzeit erwiesen. PEG ist ein nicht immunogenes, lineares, ungeladenes Polymer mit drei Wassermolekülen pro Molekül Äthylenoxid, so daß sich die hydrodynamischen Eigenschaften der konjugierten Moleküle dramatisch ändern können (Maxfield et al., Polymer, 16:505-509 (1975); Bailey, F.E., et al, in: Nonionic surfactants [Schick, M.J., Hrsg.] S. 794-821, 1967). Mehrere therapeutisch verwendete Enzyme wurden mit dem Ziel einer wirksamen Erhöhung der In-vivo-Halbwertzeit mit PEG verknüpft (Abuchowski, A. et al., J. Biol. Chem. 252:3582-3586, 1977; Abuchowski, A. et al., Cancer Biochem. Biophys., 7:175-186, 1984). Die PEG-Verknüpfung von IL-2 (Interleukin-2) soll nicht nur dessen Zirkulations-Überlebensdauer verlängern, sondern auch dessen Potenz erhöhen (Katre, N.V. et al., Proc. Natl. Acad Sci., 84:1487-1491 (1987); Goodson, R. et al., Bio/Technology, 8:343-346, 1990). Für die PEG-Verknüpfung anderer Moleküle ist eine Verminderung der Immunogenität und der Toxizität mitgeteilt worden (Abuchowski, A. et al., J. Biol. Chem., 252:3578-3581, 1977).

IL-4 kann auch in Mikrokapseln eingeschlossen werden, hergestellt z.B. durch Coacervationstechniken oder durch "Grenzschicht-Polymerisierung" ("interfacial polymerization") (z.B. Hydroxymethylcellulose- oder Gelatine-Mikrokapseln und Poly-[Methylmethacylat]-Mikrokapseln) in kolloidalen Arzneistoff-Freigabesystemen (z.B. Liposome, Albumin-Mikrosphären, Mikroemulsionen, Nano-Partikel und Nanokapseln) oder in Makroemulsionen. Solche Techniken werden in Remington's Pharmaceutical Sciences, 16. Auflage, Osol, A., Hrsg. (1980), genannt.

IL-4-Präparate sind auch bei der Antikörpergewinnung als Standards für IL-4-Assays (z.B. durch Markierung von IL-4 zur Verwendung als Standard in einem Radioimmunassay, einem enzymgekoppelten Immunassay oder in einem Radiorezeptorassay), in Affinitäts-Reinigungstechniken und in Rezeptorbindungs-Assays (vom kompetitiven Typ) bei Markierung mit Radioiod, Enzymen, Fluorophoren, "spin labels" usw. geeignet.

Da die Voraussage der Eigenschaften einer IL-4-Variante schwierig ist, ist es verständlich, daß ein gewisses "Screening" der erhaltenen Variante notwendig ist, um die optimale Variante zu erreichen. So wird z.B. eine Änderung im immunologischen Charakter des IL-4-Moleküls, z.B. die Affinität für einen bestimmten Antikörper, durch einen kompetitiven Immunassay gemessen. Die Variante wird auf Veränderungen bei der Verminderung oder Verstärkung ihrer Aktivität - verglichen mit der im gleichen Assay für das nativen IL-4 festgestellten Aktivität - überprüft. Andere potentielle Veränderungen der Protein- oder Polypeptid-Eigenschaften - wie z.B. der Redox- oder der thermischen Stabilität, der Hydrophobizität, der Empfindlichkeit gegenüber proteolytischem Abbau, der Stabilität in der rekombinanten Zellkultur oder im Plasma oder auch der Tendenz, mit "Carriern" oder zu Multimeren zu aggregieren - werden durch Methoden bestimmt, die Stand der Technik sind.

### THERAPEUTISCHE FORMULIERUNGEN UND VERABREICHUNG VON IL-4

Die erfindungsgemäßen Verbindungen inhibieren entweder Interleukin-4-vermittelte Prozesse oder konkurrieren mit dem hIL-4. Sie eignen sich deshalb zur Behandlung von überschießenden bzw. fehlgesteuerten Immunreaktionen und Autoimmunerkrankungen. Dazu zählen auch Immundefizienzen sowohl primärer als auch sekundärer Art. Darüber hinaus kann der Antagonist sowohl bei Transplantationen als auch bei der pallitativen Therapie von Tumorerkrankungen eingesetzt werden. Dazu gehören beispielsweise:
- Allergien
   (Blockierung der Primär- und der IgE vermittelten
   Antwort; Desensibilisierung bei bekannter Allergie; Atopische Erkrankungen; Linderung bei Asthma Anfällen; Hyper IgE Syndrom).
- Transplantationen
   (Reduktion der HLA-DR Expression bei
   Organtransplantation, Suppression der GVHD, Einsatz beim Purgen von Knochenmark)
- Leukämien und solide IL-4 Rezeptor exprimierende
   Tumoren
   (Reduktion einer überschießenden autokrinen IL-4
   Produktion; Hemmung des Tumorwachstums)
- Gegenregulation bei Überproduktion von Thrombozyten
- Therapie von Gerinnungsstörungen
   (via Monozytenblock)
- Verwendung bei Störungen des Lipidstoffwechsels
- Korrektur bei Störungen des Kohlehydrathaushalts
- Verbesserung des Immunstatus bei Infektionen
   (Sepsis).

Aufgrund seiner guten Wasserlöslichkeit kann das IL-4-Mutantenprotein sowohl systemisch als auch lokal d.h. topisch eingesetzt werden, u.a. inhalativ als Spray. Auch eine Formulierung als Depotpräparat ist möglich. Bei allen Therapieformen ist sowohl an eine Kurzzeittherapie als auch an eine Dauertherapie zu denken.

Therapeutische Formulierungen des IL-4-Antagonisten werden zur Lagerung dadurch zubereitet, daß der IL-4-Antagonist nach Erreichen des gewünschten Reinheitsgrades mit physiologisch akzeptablen "Carriern", Hilfsstoffen oder Stabilisatoren (Remington's Pharmaceutical Sciences, a.a.o.) in Form eines Lyophilisats oder von wässrigen Lösungen gemischt wird. Akzeptable "Carrier", Hilfsstoffe oder Stabilisatoren sind für die Empfänger bei den angewendeten Dosierungen und Konzentrationen nicht toxisch; dazu gehören Puffer wie Phosphat, Zitrat und andere organische Säuren; Antioxidantien wie z.B. Ascorbinsäure; niedermolekulare Polypeptide (weniger als etwa 10 Reste), Proteine wie Serumalbumin, Gelatin oder Immunglobuline; hydrophile Polymere wie Polyvinylpyrrolidon; Aminosäuren wie Glycin, Glutamin, Asparagin, Arginin oder Lysin; Monosaccharide, Disaccharide und andere Kohlenhydrate, z.B. Glukose, Mannose oder Dextrine; Chelatbildner wie EDTA; Zuckeralkohole wie Mannitol oder Sorbitol; salzbildende Gegenionen wie Natrium und/oder nicht-ionische oberflächenaktive Stoffe wie Tween, Pluronics oder Polyethylenglykol (PEG).

Ein IL-4-Antagonist zur in-vivo-Anwendung muß steril sein Dies wird leicht erreicht durch Filtration über sterile Membranfilter, entweder vor oder nach der Lyophilisierung und Rekonstitution. Gelagert wird der IL-4-Antagonist normalerweise in lyophilisierter Form oder in Lösung.

Geeignete Beispiele für Zubereitungen mit verzögerter Freisetzung sind z.B. semipermeable Matrizen aus festen hydrophoben Polymeren, die das Protein enthalten; bei diesen Matrizen handelt es sich um geformte Artikel ("shaped articles"), z.B. Filmtabletten oder Mikrokapseln. Beispiele für Matrizen mit verzögerter Freisetzung sind Polyester, Hydrogele [z.B. Poly(2-hydroxyethylmethacrylat) - beschrieben von Langer et al., J. Biomed. Mater. Res., 15:167-277 [1981] und Langer, Chem. Tech., 12:98-105 [1982] -oder Poly(vinylalkohol)], Polyactide (US-Pat. Nr. 3,773,919, EP 58,481), Copolymere aus L-Glutaminsäure und Gamma-ethyl-L-glutamat (Sidman et al., Biopolymers, 22:547-556 [1983]), nicht abbaubares Ethylen-vinyl-acetat (Langer et al., a.a.o.), abbaubare Milchsäure-Glykolsäure-Copolymere wie Lupron DepotTM (injizierbare Mikrosphären aus Milchsäure-Glykolsäure-Copolymer und Leuprolidazetat) und Poly-D-(-)-3-hydroxybuttersäure (EP 133,988). Während Polymere wie Ethylenvinylacetat und Milchsäure-Glykolsäure die Freisetzung der Moleküle über mehr als 100 Tage ermöglichen, erfolgt die Freisetzung der Proteine bei einigen Hydrogelen über kürzere Zeiträume. Verweilen gekapselte Proteine über längere Zeiträume im Körper, so können sie durch Feuchtigkeit bei 37 C denaturieren oder aggregieren, woraus sich ein Verlust an biologischer Wirksamkeit und mögliche Veränderungen in der Immunogenität ergeben. Zur Stabilisierung der Proteine lassen sich je nach dem in Frage kommenden Mechanismus sinnvolle Strategien entwickeln. Stellt sich z.B. heraus, daß der Mechanismus, der zur Aggregation führt, auf einer intermolekularen S-S-Brückenbildung durch Thiodisulfid-Austausch beruht, läßt sich die Stabilisierung durch Modifizierung der Sulfhydrylreste, Lyophilisierung aus sauren Lösungen, Kontrolle des Flüssigkeitsgehaltes, Verwendung geeigneter Zusatzstoffe und Entwicklung spezieller Polymer-Matrix-Zusammensetzungen erreichen.

Zu den Formulierungen eines IL-4-Antagonisten mit verzögerter Freisetzung gehören ebenso in Liposomen eingeschlossene IL-4-Antagonisten. IL-4-Antagonisten enthaltende Liposome werden mit per se bekannten Methoden hergestellt: DE 3,218,121; Epstein et al., Proc. Natl. Acad. Sci. USA, 82:3688-3692 (1985); Hwang et al., Proc. Natl. Acad. Sci. USA, 77:4030-4034 (1980); EP 52,322; EP 36,676; EP 88,046; EP 143,949; EP 142,641; japanische Patentanmeldung 83-118008; US-Pat. Nr. 4,485,045 und 4,544,545; sowie EP 102,324. Die Liposome sind in der Regel vom kleinen (etwa 200-800 Angström) unilamellaren Typ mit einem Lipidgehalt von mehr als etwa 30 Mol-% Cholesterol, wobei der Anteil für die optimale IL-4-Antagonisten jeweils angepaßt wird. Liposome mit einer verlängerten Zirkulationszeit werden im US-Pat. Nr. 5,013,556 offengelegt.

Eine weitere Anwendung der Erfindung betrifft den Einbau von IL-4-Antagonisten in "geformte Artikel". Diese können zur Modulierung oder Verhinderung des Auftretens eines Schocks eingesetzt werden.

### Beispiel 1

### Entfernung potentieller N-Glykosilierungsstellen in hIL-4-Mutantenproteinen

In der natürlichen hIL-4-Aminosäuresequenz sind zwei Asparagin-gekoppelte Glykosylierungsstellen in den Aminosäure-Positionen 38 und 105 vorhanden. Die entsprechenden Codons im Strukturgen können gegen solche für Asparaginsäure ausgetauscht werden. Dadurch unterbleibt bei Expression des Gens in Hefestämmen eine N-Glykosylierung des resultierenden hIL-4-Mutantenproteins.

Die Durchführung der beiden Codon-Austausche ("site-directed mutagenesis") im Strukturgen für hIL-4-Mutantenproteine erfolgte nach der Methode von Deng und Nickoloff [Anal. Biochem. 200:81 (1992)] unter Verwendung des Klonierungs-Vektors pUC18. Die synthetischen Oligonucleotide, die für die Änderung des Strukturgens erforderlich waren, hatten folgende Sequenzen:
a) für den Austausch Asparagin gegen Asparaginsäure in Position 38:
b) für den Austausch Asparagin gegen Asparaginsäure in Position 105:

Die in den angegebenen Nukleotidsequenzen unterstrichenen Positionen markieren die Codons für Asparaginsäure.

Durch DNA-Sequenzierung wurde der Codon-Austausch in der Nukleotidsequenz bestätigt. Das geänderte Strukturgen wurde in Hefe-Expressionsvektoren inseriert und in geeigneten Stämmen zur Expression gebracht.

### Beispiel 2

### Insertion einer Aminosäure in der Position (+ 2) zur Herstellung eines IL-4 Muteins ohne N-terminalem Methionin in E. coli

Zur Herstellung eines IL-4-Muteins, dem das N-terminale Methionin fehlt, wurde in der Position (+ 2) eine Aminosäure eingeführt, die zur Abspaltung des N-terminalen Methionins in E. coli durch eine spezifische Methionin Aminopeptidase führt (Flinta et al., Eur. J. Biochem 154, 193 - 196, 1986). Zu diesem Zweck wurde der Vektor RPR9-IL4-Y 124D (Anlage 1) mit den Restriktionsendonucleasen XhoI und BamHI geschnitten. Das hierbei entstehende ca. 450 Bp lange DNA Fragment, das die Sequenzinformationen für das IL4Y124D Gen und ein kurzes (ca. 50 Bp langes) Fragment aus der atpE Region des Vektors trägt, wurde über Agarosegelektrophorese gereinigt und in den mit SalI und BamHI geschnittenen Vektor M13mp18 umkloniert. Einzelsträngige DNA wurde hergestellt und einer 'in vitro Mutagenese Reaktion' mit folgendem Oligonukleotid unterzogen:

Durch diese Mutagenese wird in der Position (+ 2) des IL4Y124D Gens die Aminosäure Alanin (Codon GCC) eingefügt. Außerdem wird am 5'-Ende des Gens eine NcoI Schnittstelle (CCATGG) eingeführt, um das anschließende Screening und die Klonierung in einem Expressionsvektor zu erleichtern. Das 'Screening' der Plaques erfolgte durch eine Restiktionsanalyse ausgehend von doppelsträngiger M13 RF DNA (Replikative Form). Positive Klone konnten durch einen Restritionsverdau mit den Enzymen NcoI und BamHI identifiziert werden. Die korrekte Sequenz wurde zusätzlich durch eine Sequenzierung bestätigt.

Ein ca. 400 Bp langes DNA Fragment wurde mit Ncol und BamHI aus einem ausgewählten M13mp18 Klon herausgeschnitten, über Agarosegelelektrophorese gereinigt und in den ebenfalls mit Ncol und BamHI geschnittenen Vektor pTrc99A (kommerziell erhältlich von Pharmacia P-L Biochemicals) kloniert. Mit dem aus dieser Klonierung resultierenden Vektor pAPH100 (IL4Y125D) wurden E. coli Zellen (TG1) transformiert und auf Ampicillin-haltigem Nährboden selektioniert. Die Expression und Reinigung des Proteins führte zu einem IL-4 Mutein, dem das N-terminale Methionin fehlt.

### Beispiel 3:

### Fermentation der Hefezellen

### Nährlösungen:

Zur Kultivierung der hIL4-Mutantenproteine exprimierenden Hefezellen wurden die folgenden Nährlösungen verwendet:

| | **Nährlösung** | |
|---|---|---|
| **Einsatzstoff** | **SD2** | **Sc6** |
| Bacto Yeast Nitrogen Base | 6,7 g/l | - |
| Difco Hefeextrakt | - | 20,0 g/l |
| Glucose | 20,0 g/l | 5,0 g/l |
| KH₂PO₄ | 6,7 g/l | 1,4 g/l |
| (NH₄)₂SO₄ | - | 2,0 g/l |
| MgSO₄ x 7 H₂O | - | 0,5 g/l |
| Antischaummittel PPG 2000 | - | 0,1 g/l |

Die Einsatzstoffe wurden in demineralisiertem Wasser angesetzt und der pH-Wert auf pH 5,5 eingestellt. Die Sterilisation erfolgte für 20 min bei 121°C. Glucose wurde in 1/5 des erforderlichen Volumens in demineralisiertem Wasser gelöst, getrennt sterilisiert und nach dem Abkühlen zur übrigen Nährlösung gegeben.

### Stammkonserven:

Stammkonserven aller Hefetransformanten wurden durch Abfüllung von 2-ml-Aliquots einer Vorkultur und Lagerung in flüssigem Stickstoff angelegt.

### Vorkulturen:

Die Vorkulturfermentationen wurden in 1-Ltr.-Schüttelkolben, gefüllt mit 200 ml SD2-Nährlösung durchgeführt. Die Beimpfung erfolgte mit einer Stammkonserve oder mit einer Einzelkolonie von einer SD2-Agarplatte. Die Kulturen wurden unter ständigem Schütteln für 2-3 Tage bei 26-30°C inkubiert.

### Hauptkulturfermentationen:

Die Hauptkulturfermentationen wurden unter Verwendung von 10-Ltr-Rührkesselfermentern in Sc6-Nährlösung durchgeführt. Die Beimpfung erfolgte mit 3-5 Vol% einer Vorkultur, wobei vor der Beimpfung die Biomasse aus der Vorkultur abzentrifugiert und in Sc6-Medium resuspendiert wurde. Die Fermentationsbedingungen für die 10-Ltr.-Hauptkultur waren wie folgt:

| | |
|---|---|
| Temperatur | 26-30°C |
| Rührerdrehzahl | 600 rpm |
| Belüfungsrate | 0,5 vvm |
| pH-Sollwert | 5,5 (Korrektur mit 5 N NaOH und 5 N H₂SO₄) |

Ab einer Fermentationszeit von 5 Std. wurden die Kulturen kontinuierlich mit Glucose und Hefeextrakt gefüttert. Die Fütterungsrate wurde über den Respiratorischen Quotienten (RQ-Wert) der Kultur geregelt. Der RQ-Sollwert betrug 1,0. Die Fütterlösung hatte folgende Zusammensetzung:

| | |
|---|---|
| Glucose | 500 g/l |
| Difco-Hefeextrakt | 75 g/l |

Die Bestandteile wurden getrennt, in demineralisiertem Wasser gelöst und für 20 min bei 121°C sterilisiert. Nach dem Abkühlen wurden beide Lösungen vereinigt.

Bei Verwendung des induzierten Gal10-Promotors oder eines Derivats des Gal10-Promotors erfolgte die Induktion durch Wechsel des Kohlenhydrats in der Futterlösung von Glucose (500 g/l) zu Galaktose (500 g/l). Die weitere Kontrolle der Fütterungsrate erfolgte dann nicht über den RQ-Wert. Die Fütterungsrate wurde manuell auf den doppelten Wert der Fütterungsrate zum Zeitpunkt der Induktion eingestellt. Die Induktion des Gal10-Promotors erfolgte üblicherweise nach etwa 48 Std. Fermentationsdauer.

### Zellernte:

Nach Beendigung der Fermentation (80-120 Std.) wurde der Fermenterinhalt auf 10-15°C abgekühlt und im Falle der intrazellulären Expression die Hefezellen durch Standard-Zentrifugationstechniken (z.B. Becherzentrifuge) geerntet. Die nach der Zentrifugation erhaltene Zellmasse wurde durch direktes Eintropfen in flüssigen Stickstoff cryopelletisiert und bei -80°C gelagert. Die Produktaufarbeitung erfolgte aus der so behandelten Biomasse. Bei Sekretion des heterologen Proteins in die Kulturbrühe erfolgte eine Abtrennung der Hefezellen aus der Kulturbrühe durch Standard-Zentrifugationstechniken (z.B. Becherzentrifuge) oder mittels Querstrom-Mikrofiltration (z.B. Filtron-Minisette-System). Falls erforderlich wurde die Kulturbrühe sterilfiltriert. Die weitere Produktaufarbeitung erfolgte aus der zelllfreien Kulturbrühe.

### Beispiel 4

### Fermentation von E. coli

### Nährlösungen:

Die Kultivierung der hIL4-Mutantenproteine exprimierenden E.coli-Transformanten erfolgte in LB-Nährlösung folgender Zusammensetzung:

| | |
|---|---|
| Bacto Tryton | 10 g/l |
| Bacto Hefeextrakt | 5 g/l |
| NaCl | 10 g/l |

Die Bestandteile wurden in deionisiertem Wasser gelöst und für 20 min bei 121°C sterilisiert. Vor der Beimpfung wurde der Nährlösung ein zur Selektion der Transformanten geeignetes Antibiotikum (z.B. 100 mg/l Na-Ampicillin oder 50 mg/l Kanamycinsulfat in Abhängigkeit von dem im Vektor verwendeten Selektionsmarker) steril zugefügt.

### Stammkonserven:

Stammkonserven aller E. coli-Transformanten wurden durch Abfüllung von 2 ml-Aliquots einer Vorkultur und Lagerung in flüssigem Stickstoff angelegt.

### Vorkulturen:

Die Vorkulturfermentationen wurden in 1 Ltr-Schüttelkolben, gefüllt mit 200 ml LB-Nährlösung durchgeführt. Die Beimpfung erfolgte mit einer Stammkonserve oder mit einer Einzelkolonie von einer LB-Agarplatte. Die Kulturen wurden unter ständigem Schütteln für 12 - 18 Std bei 30°C inkubiert.

### Hauptkulturfermentationen:

Die Hauptkulturfermentationen wurden unter Verwendung von 10 Ltr.-Rührkesselfermentern in LB-Nährlösung durchgeführt. Die Beimpfung erfolgte mit 1-5 Vol% einer Vorkultur, wobei vor der Beimpfung die Biomasse aus der Vorkultur abzentrifugiert und in frischem LB-Medium resuspendiert wurde. Die Fermentationsbedingungen für die 10 Ltr.-Hauptkultur waren wie folgt:

| | |
|---|---|
| Start-Temperatur | 30°C (bei Verwendung temperaturinduzierbarer Promotoren) 37°C (bei Verwendung IPTG-induzierbarer Vektoren) |
| Rührerdrehzahl | 500 rpm |
| Belüftungsrate | 0,5 vvm |

Zur Verfolgung des Biomassenwachstums wurden im Abstand von ca. 1 Std. aus der Kulturbrühe sterile Proben entnommen und die optische Dichte bei 600 nm (OD600) bestimmt. Bei Erreichen einer OD600 von 0,8 - 1,2 erfolgte die Induktion der Kulturen. In Abhängigkeit vom gewählten Promotor wurde wie folgt induziert:

| | |
|---|---|
| Temperaturinduktion | Erhöhung der Fermentationstemperatur von 30°C auf 42°C |
| IPTG-Induktion | Sterile Zugabe von Isopropyl-β-D-thiogalactopyranosid (IPTG) ad 0,4 mM |

Die Induktionszeit betrug typischerweise 4 - 8 Std.

### Zellernte:

Nach Beendigung der Fermentation (6 - 14 Std.) wurde der Fermenterinhalt auf 10-15°C abgekühlt und die Bakterienzellen durch Standard-Zentrifugationstechniken (z.B. Becherzentrifuge) geerntet. Die nach der Zentrifugation erhaltene Zellmasse wurde gegebenenfalls im eingefrorenen Zustand zwischengelagert. Die Produktaufarbeitung erfolgte aus der so gewonnenen Biomasse.

### Beispiel 5

### Expression von Interleukin -4-Mutantenproteinen in Hefezellen unter Verwendung konstitutiver Promotoren

Hefetransformanten mit einem Expressionsvektor enthaltend ein für ein hIL-4-Mutantenprotein kodierendes Gen und einen konstitutiven Promotor (z.B. Alpha-Mating-Faktor-Promotor, GAPDH-Promotor, TPI-Promotor) wurden im 10 Ltr.-Maßstab bei 28°C kultiviert. Während der Fermentation erfolgte die qualitative Prüfung auf Expression des hlL-4-Mutantenproteins mittels SDS-PAGE. Die Fermentationsdauer betrug 96 Std. Die bei Fermentationsende erzielte Biomassekonzentration lag ei 27 g/l TG. Nach Abtrennung der Zellen durch Zentrifugation (15 min, 6.500 x g, 4°C) und Sterilfiltration erfolgte die Produktaufarbeitung aus der zellfreien Kulturbrühe.

### Beispiel 6

### Expression von Interleukin-4-Mutantenproteinen in Hefezellen unter Verwendung induzierbarer Promotoren

Hefetransformanten mit einem Expressionsvektor enthaltend ein für ein hIL-4-Mutanatenprotein kodierendes Gen und einen induzierbaren Promotor (z.B. Gal10-Promotor oder ein Derivat des Gal10-Promotors) wurden im 10 Ltr. Maßstab bei 28°C kultiviert. Nach einer Fermentationsdauer von 48 Std. erfolgte die Induktion durch Wechsel des in der Futterlösung verwendeten Kohlenhydrats von Glucose nach Galaktose. Während der Fermentation erfolgte die qualitative Prüfung auf Expression des hIL-4-Mutantenproteins mittels SDS-PAGE. Die Fermentationsdauer betrug 96 Std. Die bei Fermentationsende erzielte Biomassekonzentration betrug 24 g/l TG. Nach Abtrennung der Zellen und Sterilfiltration erfolgte die Produktaufarbeitung aus der zellfreien Kulturbrühe.

Analog zu diesem Verfahren können auch andere induzierbare Promotoren zur Expression von hIL-4-Mutantenproteinen eingesetzt werden. In Abhängigkeit von der Art des gewählten Promotors muß eine geeignete Induktionstechnik eingesetzt werden.

### Beispiel 7

### Expression von Interleukin-4-Mutantenproteinen in E. coli unter Verwendung induzierbarer Promotoren

E.coli-Transformanten mit einem Expressionsvektor enthaltend ein für ein hIL-4-Mutantenprotein kodierendes Gen und einen temperaturinduzierbaren Promotor (z.B. λpL-Promotor oder ein Derivat des λpL-Promotors) wurden im 10 Ltr. Maßstab in LB-Nährlösung kultiviert. Die Selektion der vektorhaltigen Zellen erfolgte durch Zuabe von 100 mg/l Na-Ampicillin zur LB-Nährlösung ( = LB+ Amp-Nährlösung). Die Beimpfung der Hauptkulturansätze erfolgte mit 5 Vol% einer 14 Std. alten in LB+Amp-Nährlösung kultivierten Vorkultur. Die Fermentationstemperatur betrug bei Beginn der Fermentation 30°C und wurde zur Induktion des temperatursensitiven Promotors nach Erreichen einer OD600 von 0,8 - 1,2 auf 42°C erhöht. Während der Fermentation erfolgte die qualitative Prüfung auf Expression des hIL-4-Mutantenproteins mittels SDS-PAGE. Nach einer Induktionsdauer von 4 - 6 Std. erfolgte die Beendigung der Fermentation durch Kühlung der Kulturbrühe auf 10-15°C. Die bei Fermentationsende erzielte Biomassekonzentration betrug ca. 5 g/l FG. Die E. coli-Zellen wurden durch Zentrifugation in der Becherzentrifuge geerntet (15 min., 6500 x g, 4°C) und die Zellmasse durch direktes Eintropfen in flüssigen Stickstoff cryopelletisiert. Die weitere Lagerung der so tiefgefrorenen Biomasse erfolgte bei -80°C. Die Produktaufarbeitung erfolgte aus der so behandelten Biomasse.

Analog zu diesem Verfahren können auch andere induzierbare Promotoren zur Expression von hIL-4-Mutantenproteinen in E. coli eingesetzt werden. In Abhängigkeit von der Art des gewählten Promotors muß eine geeignete Induktionstechnik verwendet werden.

### Beispiel 8

### Aufarbeitung eines IL-4-Mutantenproteins

### Zellaufschluß und Isolierung der "inclusion bodies"

25 g E. coli Feuchtmasse aus Beispiel 7 wurden in 200 ml Puffer (0.1 M Phosphatpuffer, pH 7,3, 0.1% Triton, 1 mM EDTA, 1 µg/ml Pepstatin) aufgenommen und durch Ultraschall (Branson Sonifier B 15) aufgeschlossen. Die das Produkt enthaltenden "inclusion bodies" wurden mittels Zentrifugation (35.000 x g, 20 min.) gewonnen und im Aufschlußpuffer,der zusätzlich 4 M Harnstoff enthielt, gewaschen.

### Solubilisierung und Sulfitolyse des Produkts

Die gewaschenen "inclusion bodies" wurden in 125 ml Puffer (0,2 M Tris, pH 8,1, 8 M Guanidinhydrochlorid) solubilisiert. 4 g Natriumsulfit und 2 g Kaliumtetrathionat wurden zugegeben, und die Reaktionsmischung 2 h gerührt. Ungelöste Bestandteile wurden nach Beendigung der Reaktion durch Zentrifugation entfernt (35.000 x g, 20 min.).

### Gelfiltration

Der Überstand wurde auf eine Gelfiltrationssäule (Sephacryl-S-300 HR, Pharmacia, 10 x 90 cm) aufgetragen und in PBS-Puffer, der 6 M Guanidinhydrochlorid enthielt, mit einer Flußrate von 280 ml/h gelfiltriert. Produkthaltige Fraktionen wurden mittels SDS-PAGE identifiziert und vereinigt.

### Renaturierung

Zur Reduktion der Moleküle wurde β-Mercaptoethanol (Endkonzentration 15 mM) hinzugegeben. Nach zwei Stunden Inkubation bei Raumtemperatur wurde der Ansatz 5fach mit Wasser verdünnt und 3-4 Tage gegen Puffer (3 mM NaH₂PO₄, 7 mM Na₂HPO₄, 2 mM KCl, 120 mM NaCl) dialysiert.

### Konzentrierung

Das dialysierte Material wurde mit Essigsäure auf pH 5 eingestellt und die leitfähigkeit durch Zugabe von Wasser auf ≤ 10 mS/cm verringert. 50 ml CM-Sepharose-FF (Pharmacia), equilibriert mit 25 mM Ammoniumacetat, pH 5,0, wurden unter Rühren zum Ansatz gegeben. Ungebundenes Material wurde abfiltriert, und das Gel in eine Säule gefüllt. Das Produkt wurde mit einem linearen Gradienten von 0-1 M NaCl in 25 mM Ammoniumacetat, pH 5,0, mit einer Flußrate von 300 ml/h eluiert. Produkthaltige Fraktionen wurden mittels SDS-PAGE oder analytischer RP-Chromatographie identifiziert.

### Endreinigung

Der Pool der CM-Sepharose wurde auf eine mit 0,1% TFA equilibrierten Vydac C-4 Säule (1 x 25 cm, 10 µm) aufgetragen und mit einem steigenden Acetonitrilgradienten eluiert. Fraktionen, die Reinprodukt enthielten, wurden vereinigt und lyophilisiert.

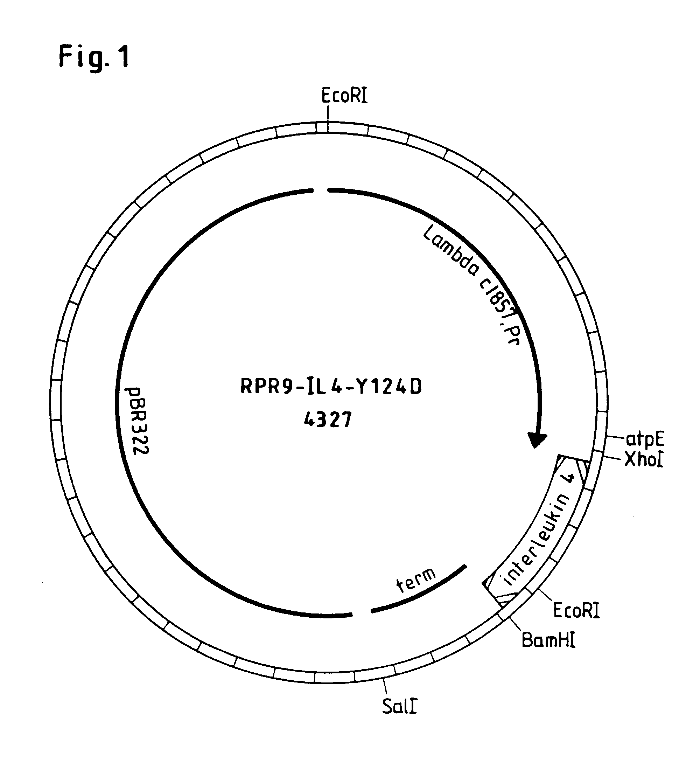

## Patentansprüche

1. Humane lnterleukin-4-Mutantenproteine als Antagonisten oder partielle Agonisten des humanen Interleukin 4, dadurch gekennzeichnet, daß sie neben Austauschen in den Positionen 121, 124 oder 125 zusätzlich eine Insertion einer Aminosäure zwischen dem N-terminalen Methionin und dem natürlichen N-terminus des hlL-4 Mutantenproteins haben.

2. Humane lnterleukin-4-Mutantenproteine gemäß Anspruch 1, wobei die insertierte Aminosäure Gly, Pro, Ser, Thr oder Val ist.

3. Humane lnterleukin-4-Mutantenproteine gemäß Anspruch 1, wobei die insertierte Aminosäure Ala ist.

4. Humane lnterleukin-4-Mutantenproteine aus der Gruppe
Ala(-1)-Tyr(124)Asp,
Ala(-1)-Arg(121)Asp-Tyr(124)Asp,
Ala(-1)-Arg(121)Asp-Tyr(124)Asp-Ser(125)Asp,
Ala(-1)-Tyr(124)Asp-Ser(125)Asp oder
Ala(-1)-Arg(121)Asp-Ser(125)Asp.

5. Arzneimittel, die Substanzen gemäß den Ansprüchen 1 bis 4 enthalten.

6. Verwendung der humanen lnterleukin-4-Mutantenproteine aus den Ansprüchen 1 bis 4 zur Herstellung von Arzneimitteln.

## Claims

1. Human interleukin 4 mutant proteins as antagonists or partial agonists of human interleukin 4, characterized in that, apart from substitutions at positions 121, 124 or 125, they additionally possess an insertion of an amino acid between the N-terminal methionine and the natural N terminus of the hIL-4 mutant protein.

2. Human interleukin 4 mutant proteins according to Claim 1, wherein the inserted amino acid is Gly, Pro, Ser, Thr or Val.

3. Human interleukin 4 mutant proteins according to Claim 1, wherein the inserted amino acid is Ala.

4. Human interleukin 4 mutant proteins from the group
Ala(-1)-Tyr(124)Asp,
Ala(-1)-Arg (121)Asp-Tyr(124)Asp,
Ala(-1)-Arg(121)Asp-Tyr(124)Asp-Ser(125)Asp,
Ala(-1)-Tyr(124)Asp-Ser(125)Asp or
Ala(-1)-Arg(121)Asp-Ser(125)Asp.

5. Medicaments which comprise the substances according to Claims 1 to 4.

6. Use of the human interleukin 4 mutant proteins from Claims 1 to 4 for preparing medicaments.

## Revendications

1. Protéines mutantes de l'interleukine-4 humaine, comme antagonistes ou agonistes partiels de l'interleukine-4 humaine, caractérisées en ce qu'elles ont, en plus de l'échange en les positions 121, 124 ou 125, en outre, une insertion d'un acide aminé entre la méthionine N-terminale et l'extrémité N naturelle de la protéine mutante IL-4 humaine.

2. Protéines mutantes de l'interleukine-4 humaine suivant la revendication 1, où l'acide aminé inséré est Gly, Pro, Ser, Thr ou Val.

3. Protéines mutantes de l'interleukine-4 humaine suivant la revendication 1, où l'acide aminé inséré est Ala.

4. Protéines mutantes de l'interleukine-4 humaine parmi le groupe :
Ala(-1)-Tyr(124)Asp ;
Ala(-1)-Arg(121)Asp-Tyr(124)Asp ;
Ala(-1)-Arg(121)Asp-Tyr(124)Asp-Ser(125)Asp ;
Ala(-1)- Tyr(124)Asp-Ser(125)Asp, ou
Ala(-1)-Arg(121)Asp-Ser(125)Asp.

5. Médicaments qui contiennent les substances suivant les revendications 1 à 4.

6. Utilisation des protéines mutantes de l'interleukine-4 humaine suivant les revendications 1 à 4, pour la préparation de médicaments.


### SEQUENZPROTOKOLL

(1) ALGEMEINE INFORMATION:
   (i) ANMELDER:
      (A) NAME: Bayer AG
      (B) STRASSE: Bayerwerk
      (C) ORT: Leverkusen
      (E) LAND: Deutschland
      (F) POSTLEITZAHL: D-51368
      (G) TELEPHON: 0214/3061455
      (H) TELEFAX: 0214/303482
   (ii) ANMELDETITEL: Neue hIL4-Mutantenproteine als Antagonisten oder partielle Agonisten des humanen Interleukin 4
   (iii) ANZAHL DER SEQUENZEN: 5
   (iv) COMPUTER-LESBARE FORM:
      (A) DATENTRÄGER: Floppy disk
      (B) COMPUTER: IBM PC compatible
      (C) BETRIEBSSYSTEM: PC-DOS/MS-DOS
      (D) SOFTWARE: Patentln Release #1.0, Version #1.25 (EPA)
(2) INFORMATION ZU SEQ ID NO: 1:
   (i) SEQUENZ CHARAKTERISTIKA:
      (A) LÄNGE: 16 Basenpaare
      (B) ART: Nukleinsäure
      (C) STRANGFORM: Einzel
      (D) TOPOLOGIE: linear
   (ii) ART DES MOLEKÜLS: cDNS
   (iii) HYPOTHETISCH: NEIN
   (iii) ANTISENSE: NEIN
   (vi) URSPRÜNLICHE HERKUNFT:
      (C) INDIVIDUUM ISOLAT: synthetisch
   (xi) SEQUENZBESCHREIBUNG: SEQ ID NO: 1:
   CATGCACAAG TGCGAT 16
(2) INFORMATION ZU SEQ ID NO: 2:
   (i) SEQUENZ CHARAKTERISTIKA:
      (A) LÄNGE: 12 Basenpaare
      (B) ART: Nukleinsäure
      (C) STRANGFORM: Einzel
      (D) TOPOLOGIE: linear
   (ii) ART DES MOLEKÜLS: cDNS
   (iii) HYPOTHETISCH: NEIN
   (iii) ANTISENSE: NEIN
   (vi) URSPRÜNLICHE HERKUNFT:
      (C) INDIVIDUUM ISOLAT: synthetisch
   (xi) SEQUENZBESCHREIBUNG: SEQ ID NO: 2:
   ATCGCACTTG TG 12
(2) INFORMATION ZU SEQ ID NO: 3:
   (i) SEQUENZ CHARAKTERISTIKA:
      (A) LÄNGE: 21 Basenpaare
      (B) ART: Nukleinsäure
      (C) STRANGFORM: Einzel
      (D) TOPOLOGIE: linear
   (ii) ART DES MOLEKÜLS: cDNS
   (iii) HYPOTHETISCH: NEIN
   (iii) ANTISENSE: NEIN
   (vi) URSPRÜNLICHE HERKUNFT:
      (C) INDIVIDUUM ISOLAT: synthetisch
   (xi) SEQUENZBESCHREIBUNG: SEQ ID NO: 3:
   GCCTCCAAGG ACACAACTGA G 21
(2) INFORMATION ZU SEQ ID NO: 4:
   (i) SEQUENZ CHARAKTERISTIKA:
      (A) LÄNGE: 24 Basenpaare
      (B) ART: Nukleinsäure
      (C) STRANGFORM: Einzel
      (D) TOPOLOGIE: linear
   (ii) ART DES MOLEKÜLS: cDNS
   (iii) HYPOTHETISCH: NEIN
   (iii) ANTISENSE: NEIN
   (vi) URSPRÜNLICHE HERKUNFT:
      (C) INDIVIDUUM ISOLAT: synthetisch
   (xi) SEQUENZBESCHREIBUNG: SEQ ID NO: 4:
   GTGAAGGAAG CCGACCAGAG TACG 24
(2) INFORMATION ZU SEQ ID NO: 5:
   (i) SEQUENZ CHARAKTERISTIKA:
      (A) LÄNGE: 36 Basenpaare
      (B) ART: Nukleinsäure
      (C) STRANGFORM: Einzel
      (D) TOPOLOGIE: linear
   (ii) ART DES MOLEKÜLS: cDNS
   (iii) HYPOTHETISCH: NEIN
   (iii) ANTISENSE: NEIN
   (vi) URSPRÜNLICHE HERKUNFT:
      (C) INDIVIDUUM ISOLAT: synthetisch
   (xi) SEQUENZBESCHREIBUNG: SEQ ID NO: 5:
   CTGGAGACTG CCATGGCCCA CAAGTGCGAT ATCACC 36